# EUROPÄISCHE PATENTSCHRIFT

(11) **EP 2 988 817 B1**
(45) Veröffentlichungstag und Bekanntmachung des Hinweises auf die Patenterteilung: **16.02.2022**
(21) Anmeldenummer: 14718068.1
(22) Anmeldetag: 16.04.2014
(51) Int. Cl.: A61M 25/10

(54) **BALLONKATHETER**
BALLOON CATHETER
CATHÉTER À BALLONNET

(30) Priorität: 22.04.2013 DE 102013104029
(43) Veröffentlichungstag der Anmeldung: 02.03.2016
(73) Patentinhaber: Innora GmbH, 10115 Berlin (DE)
(72) Erfinder: HANISCH, Uli, 10961 Berlin (DE); SPECK, Ulrich, 13465 Berlin (DE)
(74) Vertreter: ETL IP Patent- und Rechtsanwaltsgesellschaft mbH
(86) Internationale Anmeldenummer: PCT/EP2014/057701
(87) Internationale Veröffentlichungsnummer: WO 2014/173748

(56) Entgegenhaltungen:
- EP-A1- 2 510 972
- EP-B1- 2 510 972
- WO-A1-95/08965
- WO-A1-96/19256
- WO-A1-2011/130679
- WO-A2-2007/054364
- US-A- 5 383 856
- US-A- 5 879 499
- US-A1- 2006 287 665
- US-A1- 2009 018 501
- US-A1- 2009 171 278

## Beschreibung

Die vorliegende Erfindung betrifft Katheter mit Ballon für medizinische Anwendungen.

Körperhöhlen und Blutgefäße unterschiedlicher Art können aus verschiedenen Gründen eine medizinische Behandlung mit mechanischen oder pharmakologischen Mitteln erfordern. Die hier dargelegte Erfindung betrifft Medizinprodukte und Methoden, die die bisherigen Behandlungsmöglichkeiten erweitern und vor allem Wandunregelmäßigkeiten, Strukturveränderungen, Entzündungen, Wandverdickungen und in der Wand lokalisierte metabolische Erkrankungen betreffen.

Die minimalinvasive Behandlung mit Kathetern hat bei zahlreichen Erkrankungen des Blutgefäßsystems und anderer Körperhöhlen die Chirurgie ersetzt oder die Wirksamkeit der Pharmakotherapie durch gezielte lokale Arzneimittelverabreichung verbessert. Durch lokale Gabe können hohe Arzneimittelkonzentrationen im erkrankten Gewebe erreicht werden ohne oder bei deutlich vermindertem Risiko unerwünschter systemischer Wirkungen. Der Schwerpunkt der Entwicklung der letzten Jahrzehnte lag auf der Wiederherstellung des normalen arteriellen Blutflusses und der Sauerstoffversorgung nachgeschalteter Gewebe im Falle von hochgradigen Arterienverengungen oder Arterienverschlüssen. Betroffen sind meist die Herzkranzgefäße, Beingefäße, Karotiden, Nierenarterien oder künstlich angelegte Passagen wie Dialyseshunts. Diese Gefäßabschnitte sind häufig fibrotisch verhärtet oder verkalkt oder durch Thromben verschlossen. Zur Wiedereröffnung gibt es zahlreiche Instrumente und Hilfsmittel, die auf ganz unterschiedliche Weise wirken. Am gebräuchlichsten sind Ballonkatheter, deren Ballone aus im Wesentlichen inelastischen Materialien wie Nylon bestehen. Die Ballone haben einen vorgegebenen Durchmesser und eine vorgegebene Länge, die sich wegen der geringen Dehnbarkeit des Materials auch unter hohem Innendruck nicht wesentlich verändern. Hohe Drücke sind erforderlich, um stark verengte Gefäßlumina zu erweitern und wieder durchgängig zu machen. Die Formstabilität der Ballone verhindert, daß die Gefäße überdehnt werden, jedenfalls dort, wo der Widerstand geringer ist als in anderen Teilen des erkrankten Gefäßes.

Um zu verhindern, daß es durch elastische Rückstellkraft der Gefäße zu einer sofortigen Wiederverengung kommt, können Stents implantiert werden.

Der auf Gefäße ausgeübte Druck und die unter hohem Druck sehr harten und steifen Ballone können zu Gefäßeinrissen und einer Ablösung von Teilen der Gefäßinnenwand führen (Dissektionen), die akut den Blutfluß behindern und auch verzögert die Bildung von Thromben verursachen können. Um Dissektionen wieder an die Gefäßwand anzulegen werden die Ballone für längere Zeit bis zu einigen Minuten inflatiert oder ebenfalls Stents implantiert. Elastische Rückstellung einer Gefäßwand und Dissektion sind sofort nach dem Eingriff erkennbar.

Andere Verfahren der Erweiterung oder Wiedereröffnung von Gefäßlumina sind Atherektomie und Laser, die langstreckig eingesetzt werden können, jedoch starke Gefäßwandverletzungen verursachen.

Im Zuge der Abheilung kann es zu einer überschießenden Gewebe-/ Narbenbildung kommen, die das Gefäßlumen einengt. Dieser Vorgang dauert gewöhnlich Monate. Er kann meist durch die lokale Verabreichung von Arzneimitteln mittels implantierter Stents oder durch die Verwendung von speziellen Ballonkathetern, die mit Arzneimitteln beschichtet sind, verhindert werden.

Nachteile der beschrieben lokalen Behandlung von verengten und verschlossenen Arterien sind die strenge Begrenzung der Behandlung auf die betroffenen Gefäßabschnitte, d.h. Gefäße, deren Durchmesser auf ≤ 50 % des ursprünglichen Durchmessers verengt sind. Erkrankte benachbarte Gefäßabschnitte ohne starke Verengung bleiben unbehandelt, um nicht durch mechanischen Schaden die reaktive Hyperproliferation zu initiieren. Solche benachbarten unbehandelten Gefäßabschnitte sind häufig unregelmäßig geformt, entzündet und weisen Auflagerungen (vulnerable plaques) auf, die aufbrechen können, thrombogeneOberflächen freisetzen und zu plötzlichen Gefäßverschlüssen führen können. Für deren Behandlung sind verschiedene, ebenfalls örtlich sehr begrenzte Maßnahmen vorgeschlagen worden, ohne daß der Nutzen einer dieser Maßnahmen bisher durch klinische Prüfungen belegt wäre. Solche Maßnahmen beinhalten die Abdeckung der erkrankten Gefäßabschnitte durch Stents (z.B. US 2009104246, US 20090030494 mit Arzneistoffen), durch Polymere oder Polymerisation (US 7,473,242, WO 2011044455, US 7846147) oder durch kontrollierte Zerstörung des Plaques (US 7753926).

Es fehlt an einer einfachen wirksamen Behandlungsmethode für diese langstreckig geschädigten Gefäßabschnitte im Zuge einer Angioplastie und/ oder Stentimplantation, die vermeidet, daß diese Gefäßabschnitte durch mechanische Verletzung längerfristig geschädigt werden. Es fehlt ebenfalls an einer einfachen Behandlung von Gefäßveränderungen an Aufzweigungen.

Mit Arzneimitteln beschichtete Ballonkatheter sind etwa in den Dokumenten US 6,616,650, US 5304121 und USP 5,102,402 beschrieben. In tierexperimentellen Untersuchungen konnte gezeigt werden, daß geeignete Beschichtungen trotz sehr kurzer Kontaktzeit des Ballons mit der Arterienwand eine das Lumen einengende Hyperproliferation nach Gefäßdilatationzu hemmen vermögen (Scheller B, Speck U, Abramjuk C, Bernhardt U, Böhm M, Nickenig G. Paclitaxel ballooncoating - a novelmethodforpreventionandtherapyofrestenosis. Circulation 2004;110:810-814). Klinisch wurde eine dauerhafte Verminderung der Gefäßverengung nach Angioplastie sehr unterschiedlicher Gefäße gezeigt (z.B. Scheller B, Clever YP, Kelsch B, Hehrlein C, Bocksch W, Rutsch W, Haghi D, Dietz U, Speck U, Böhm M, Cremers B. Longterm follow-up after treatment of coronary in-stent restenosis with a paclitaxel-coated balloon catheter. J Am Coll Cardiol Intv 2012;5:323-330). Die in den genannten Publikationen verwendeten Beschichtungen wurden unter anderem in der EP 1 372 737 beschrieben. Weitere Patentschriften beschreiben weitere mögliche Formulierungen und Verfahren, etwa die EP2170421 und US8244344.

Die in der Angioplastie gebräuchlichen Ballone bestehen aus den oben beschriebenen Gründen aus im Wesentlichen inelastischen Materialien wie Nylon. Um zum Einführen in die Arterie und zur Passage durch Verengungen des Gefäßes einen geringen Querschnitt zu erreichen, werden die Ballone vollständig entleert, gefaltet und die Falten werden um einen längs durch den Ballon verlaufenden Schaft gewickelt. Dann werden diese Ballone mit Flüssigkeit unter hohem Druck gefüllt. Sie zwingen dem Gefäßlumen ihre Form auf, d.h. das Lumen soll in dem behandelten Bereich den Durchmesser des Ballons annehmen. Dabei kommt es meist zu starken Gefäßwandverletzungen.

Katheter mit Ballonen aus elastischen Membranen, die mit geringem Innendruck mehr oder weniger weit expandiert werden können, wurden vor allem zur Fixierung von Blasenkathetern und anderen für Drainagen verwendeten Kathetern benutzt. Die dafür eingesetzte Ballonform ist annähernd rund. Der Ballon kann und soll sich demnach in alle Richtungen ausdehnen und nicht an bestimmte Gefäßwände anlehnen, um diese aufzuweiten.

Ballone aus gut dehnbaren Materialien (z. B. Polyurethan, Latex, Polyisopren) wurden auch im Zusammenhang mit der Behandlung von unregelmäßig geformten Blutgefäßen erwähnt (z.B. US 20090258049). In anderen Fällen wurden weiche Ballone für bestimmte Anwendungen bevorzugt, z. B. zum Verschluß von Zugängen zu Aneurismen (US 8066667), zur Behandlung von Bifurkationen (US 8216267), zur lokalen Verabreichung von Arzneistoffen an die Wand von unregelmäßig geformten Körperhöhlen (US 20080243103), oder auch zur Behandlung von unregelmäßigen und empfindlichen Gefäßwandstrukturen mit Arzneimitteln, die von den Ballonen freigegeben werden (US 7179251, WO 2012158944). Hierbei handelt es sich jeweils um relativ kurze Ballone, soweit angegeben mit bis zu 4 cm Länge.

Ein gravierendes Problem ist die Längenausdehnung der weichen Ballonmaterialien sowie bei längeren Ballonen die unregelmäßige, oft nur teilweise oder abschnittweise Füllung. Bei geringem Druck vergrößert sich der Durchmesser und der Ballon legt sich zumindest teilweise an der Gefäßwand an. Gleichzeitig dehnt sich der Ballon in der Länge aus. Da der Ballon proximal und distal an einem zentralen Schaft befestigt ist, führt die Längsdehnung zu einer spiraligen oder andersartigen unerwünschten Verformung des Ballons. Um dem entgegenzuwirken, können die expandierbaren Schläuche in der Längsachse vorgespannt auf den Schaft montiert werden (US 6344045,US 8066667). Beim Füllen des Lumens mit Flüssigkeit dehnt sich der Schlauch zunächst in der gewünschten Richtung auf, d.h. er vergrößert den Durchmesser ohne wesentliche Änderung seiner vorgespannten Länge. Die Vorspannung ist aber nur möglich, wenn der Katheterschaft steif ist. Dies ist u.a. unerwünscht, da Katheter durch stark gewundene Gefäße geführt werden müssen.

Eine andere Möglichkeit, eine unerwünschte Längsausdehnung zu verhindern. wurde in WO 2005025648 vorgeschlagen: In eine mehrschichtige elastische Ballonmembran werden wenig elastische Fäden oder textile Gewebe eingebettet, die die Längsdehnung verhindern oder begrenzen. Nachteil ist der komplexe Aufbau der Membran und die damit verbundene unerwünschte Dickenzunahme.

Unter hohem Innendruck ist auch bei Ballonen mit im Wesentlichen inelastischen Membranen eine gewisse Längenzunahme zu beobachten. Diese führt zu einer unerwünschten Krümmung des proximal und distal an einen unelastischen Schaft fixierten Ballons. Durch Einbau eines in der Längsrichtung elastischen Elements im Schaft zwischen den proximal und distal fixierten Ballonenden läßt sich die Krümmung des Ballons durch geringe Längenausdehnung vermeiden, da sich der Schaft ebenfalls dehnt (US 8292912).

Siehe weiterhin US 2009/018501 A1, US 5 879 499 A, US 2006/287665 A1, US 2009/171278 A1, WO 2011/130679 A1, WO 96/19256 A1.

Eine Aufgabe der vorliegenden Erfindung besteht darin, einfache und kostengünstige Katheter bereitzustellen, mit denen langstreckige Veränderungen in Gefäßen mit wechselndem Lumendurchmesser und Verzweigungen durch mechanische Glättung und lokale Arzneimittelgabe behandelt werden können. Insbesondere ist es Aufgabe der Erfindung Verformungen langer dehnbarer Katheterballone während der Insufflation durch Längsausdehnung zu verhindern oder zu beschränken.

Diese Aufgabe wird durch einen Ballonkatheter nach Anspruch 1 gelöst.

Vorteilhafte Ausführungsformen und Weiterbildungen ergeben sich aus den abhängigen Ansprüchen.

### Beschreibung der Erfindung

In dieser Beschreibung wird ein näher am Arzt liegender Bereich als proximaler Bereich bezeichnet, während das in einen Körper einzuführende Ende eines Katheters als distaler Bereich bezeichnet wird. Die Bezeichnung "Ballon" wird synonym verkürzend für Katheterballon verwendet.

Als dehnbar werden Ballon-Membranen bezeichnet, die es erlauben, bei einer Differenz zwischen Außendruck (Druck in dem den Ballon umgebenden Bereich) und Innendruck im Ballon von weniger als 2,0266 bar (2atm), d.h. bei einem Innendruck, der zwischen 0,10133 bar (0,1 atm) und weniger als 2,0266 bar (2 atm) höher als der Außendruck ist, den Durchmesser des Ballons senkrecht zur Längsachse, insbesondere in einem mittleren Bereich des Ballons, d.h. nicht im Bereich der Ballonschultern am distalen und proximalen Ballonende, durch Dehnung der Membran auf mindestens das 2,5 fache zu vergrößern. Dies trifft beispielsweise zu wenn ein Ballonquerschnitt von 2 mm im ungefaltet spannungsfreien Zustand (Innendruck im Ballon = Außendruck) bei einem Innendruck von <2,0266 bar (2 atm) über dem Außendruck auf 5 mm oder mehr erweitert wird. Als hochdehnbar werden Membranen bezeichnet, die es erlauben den Ballondurchmesser unter gleichen Bedingungen auf mindestens das 5,0 fache zu vergrößern, im Beispiel also sich auf ≥10 mm erweitern lassen. Dabei vergrößert sich die Oberfläche der Ballone annähernd entsprechend durch Dehnung, d.h. eine Durchmesservergrößerung auf das 2,5fache entspricht einer Oberflächenvergrößerung auf das 2,5fache etc.. Ergänzend werden als elastisch bzw. hochelastisch dehnbare oder hochdehnbare Membranen bezeichnet, die bei Druckminderung wieder die annähernd gleiche Oberfläche erreichen wie vor der Dehnung. "Annähernd gleich" bedeutet, dass die Oberfläche des Ballons nach der Dehnung nur um maximal 25% größer als vor der Dehnung ist. Unter dem Durchmesser des Ballons (=Ballondurchmesser) wird hier und nachfolgend immer der Durchmesser senkrecht zur Längsachse des Ballons verstanden.

Die vorliegende Erfindung beschreibt in einer Ausführung einen Ballonkatheter mit einem Ballon und einem Katheterschaft, welcher ein Drahtlumen bildet, das zum Führen eines Führungsdrahtes ausgebildet ist, sowie ein Flüssigkeitslumen bildet, über welches dem Ballon eine Flüssigkeit zuführbar ist. Der Ballon ist an einer ersten, proximalen Befestigungsstelle an einem ersten Katheterschaftelement befestigt. Ferner ist der Ballon an einer zweiten, distalen Befestigungsstelle an einem zweiten Katheterschaftelement befestigt, wobei die erste Befestigungsstelle gegenüber der zweiten Befestigungsstelle entlang einer Längsachse des Katheterschafts bewegbar ist. Der Ballon umgibt einen Abschnitt des Katheterschafts, welcher sich zwischen der ersten und zweiten Befestigungsstelle erstreckt. Dabei ist vorgesehen, daß der vom Ballon umgebene Abschnitt des Katheterschafts mit sich ändernder Längenausdehnung des Ballons unterschiedliche Bereiche des Katheterschafts umfasst. Somit kann erfindungsgemäß eine Anpassung der Länge des Ballons ohne unerwünschte Verformung und ohne aufwendige Anpassung von Katheterschaftelementen erfolgen. Dabei kann insbesondere vorgesehen sein, daß durch Längenausdehnung des Ballons Bereiche des Katheterschafts, insbesondere eines inneren Schlauchs und/oder eines das Drahtlumen bereitstellenden Schlauchs, vom Ballon umgeben werden, die vor der Längenausdehnung nicht vom Ballon umgeben waren. Eine Längenausdehnung des Ballons kann durch manuelle oder mechanische Verschiebung einer oder beider Befestigungsstellen und/oder manuelle oder mechanische Verschiebung oder elastisches Ausdehnen des ersten und/oder zweiten Katheterschaftelements erfolgen. Alternativ oder zusätzlich kann eine Längenausdehnung durch Versorgen des Ballons mit einer Flüssigkeit über das Flüssigkeitslumen erfolgen. Dabei kann beispielsweise das zweite Katheterschaftelement beim Füllen des Ballons durch den in der Längsachse wirkenden Druck gegenüber dem ersten Katheterschaftelement bewegt werden. Durch die relative Bewegung der Katheterschaftelemente zueinander können jeweils die erste und/oder zweite Befestigungsstelle mitgenommen und relativ zueinander bewegt werden. Allgemein kann mit einer relativen Bewegung der Befestigungselemente zueinander eine Bewegung entlang einer Längsachse des Katheterschafts und/oder eineLängenänderung des zwischen ihnen aufgespannten und/oder gehaltenen Ballons verbunden sein, insbesondere eine Längenausdehnung, wenn sich die Befestigungselemente und/oder die zugeordneten Katheterschaftelemente voneinander entfernen. Das erste Katheterschaftelement und das zweite Katheterschaftelement können gegeneinander lösbar festlegbar sein und/oder selektiv gegeneinander verschiebbar sein, etwa durch eine lösbare Klemmverbindung. Das erste Katheterschaftelement kann insbesondere ein Schlauch sein, durch den der Ballon mit Flüssigkeit befüllt oder wieder entleert wird. Somit kann das Flüssigkeitslumen durch das erste Katheterschaftelement bereitgestellt oder gebildet sein. Etwa kann das erste Katheterschaftelement ein äußerer Schlauch, in welchen ein innerer Schlauch zumindest teilweise aufgenommen ist, oder ein Schlauch sein, welcher das Flüssigkeitslumen und das Drahtlumen in getrennten Lumina bereitstellt, etwa ein Doppellumenschlauch. Es kann vorgesehen sein, dass das Drahtlumen und das Flüssigkeitslumen durch aneinander befestigte Schläuche und/oder Schlauchelemente gebildet sind. Das zweite Katheterschaftelement kann insbesondere ein innerer Schlauch sein, welcher das Drahtlumen enthalten oder bilden kann. Ein solcher Schlauch kann in einem äußeren Schlauch zumindest teilweise aufgenommen sein und gegenüber diesem bewegbar sein. Alternativ kann das zweite Katheterschaftelement ein beweglich auf einem inneren Schlauch und/oder auf einem Drahtführungsschlauch, welcher etwa ein Drahtlumen enthalten oder bilden kann, gelagertes Führungselement umfassen oder als solches ausgebildet sein. Ein derartiges Führungselement kann etwa als Gleitelement und/oder ein Ring oder Schlauchstück oder Rohr ausgebildet sein.Ein Gleitelement kann allgemein in gleitendem und/oder kraftschlüssigverschiebbaren und/oder zumindest teilweise abdichtenden Kontakt mit einem es lagernden Element stehen. Der innere Schlauch oder Drahtführungsschlauch, auf welchem das Führungselement verschiebbar gelagert ist, und/oder ein fest damit verbundenes oder einstückig damit ausgebildetes Element kann als erstes Katheterschaftelement dienen oder angesehen werden. Es kann dabei vorgesehen sein, dass das zweite Katheterschaftelement distal des Flüssigkeitslumens und/oder einer Öffnung, über welche dem Ballon über das Flüssigkeitslumen Flüssigkeit zuführbar und/oder daraus entleerbar ist, angeordnet und/oder dagegen distal verschiebbar ist. Der Ballon kann an der ersten Befestigungsstelle direkt oder indirekt am ersten Katheterschaftelement befestigt sein, etwa indem er an einer Komponente befestigt ist, welche relativ zum ersten Katheterschaftelement fixiert und/oder unbeweglich ist oder auch auf der Längsachse des ersten Katheterschaftelements beweglich ist. Der Ballon kann an der zweiten Befestigungsstelle direkt oder indirekt am zweiten Katheterschaftelement befestigt sein, etwa indem er an einer Komponente befestigt ist, welche relativ zum zweiten Katheterschaftelement fixiert und/oder unbeweglich ist. Das erste Katheterschaftelement und/oder das zweite Katheterschaftelement, insbesondere ein das Drahtlumen enthaltender oder bildender Schlauch oder innerer Schlauch, können elastisch ausgebildet sein. Allgemein kann das Flüssigkeitslumen zum Befüllen und/oder Entleeren des Ballons dienen. Zum Füllen des Ballons werden physiologisch verträgliche Flüssigkeiten, meist verdünnte Röntgenkontrastmittel, bevorzugt.

Der Ballon kann eine Ballonmembran aufweisen, die aus einem oder mehreren dehnbaren Elastomeren besteht, bevorzugt aus Gummi, Latex, Polyrurethan, Isopren-basierten Polymeren oder Copolymeren, Silikon, besonders bevorzugt aus Latex, Polyurethan oder beispielsweise Styrol-Isopren-Blockcoplymeren wie ChronoPrene^{®} und am meisten bevorzugt aus Chronoprene^{®} . Chronoprene^{®} ist der Markenname der Firma Cardio Tech International, Inc., jetzt AdvanSource Biomaterials, Inc., USA für eine Mischung bzw. ein Blockcopolymer aus Polystyrol und hydriertem Polyisopren und Zusätzen zur Verbesserung der Festigkeit (Polypropylen) und Geschmeidigkeit (Mineralöl). Allgemein kann der Ballon eine elastische oder hochelastische Ballonmembran aufweisen, die sich gut auch an komplexe Strukturen anlegt.

Die Länge des Ballons im nicht ausgedehnten Zustand beträgt ≥ 10 cm, bevorzugt ≥ 15 cm und besonders bevorzugt ≥ 20 cm.

Somit können lange Gefäßabschnitte behandelt und/oder durch den Ballon abgestützt werden. Für die Anwendung in Koronararterien oder anderen kleinen Arterien mit vergleichbarer Länge wie Koronararterien können die Ballons kürzer sein. Sie weisen etwa im nicht expandierten Zustand eine Länge auf, die das mehr als 5fache, bevorzugt das mehr als 10fache und besonders bevorzugt mehr als das 15fache des Referenzdurchmessers des zu behandelnden Gefäßsegments beträgt. Der Referenzdurchmesser des Gefäßes ist in diesem Fall derjenige Durchmesser, der in einem benachbarten nicht erkrankten Segment proximal des erkrankten Segments gemessen wird.

Bei einer Weiterbildung kann der Katheterschaft als zweites Katheterschaftelement einen inneren Schlauch umfassen, welcher proximal der ersten Befestigungsstelle aus elastischem Material besteht. Insbesondere kann vorgesehen sein, daß der innere Schlauch über seine gesamte Länge und/oder in seiner Länge proximal bis zur zweiten Befestigungsstelle im Wesentlichen gleichbleibende elastische Eigenschaften aufweist. Dies ermöglicht es einerseits, die elastischen Eigenschaften über eine große Länge des inneren Schlauchs zu nutzen. Andererseits ist es nicht notwendig, einen komplexen inneren Schlauch mit unterschiedlichen Elastizitäten einzusetzen.

Es kann vorgesehen sein, daß ein als innerer Schlauch des Katheterschafts ausgebildetes zweites Katheterschaftelement proximal über eine Dichtung aus einem als äußerer Schlauch des Katheterschafts ausgebildeten ersten Katheterschaftelement herausgeführt ist und relativ zu dem äußeren Schlauch bewegbar angeordnet oder ausgebildet ist. Dies ermöglicht beispielsweise eine manuelle Verschiebbarkeit des inneren Schlauchs durch einen Arzt.

Die zweite Befestigungsstelle des Ballons kann entlang einer Längsachse eines das Drahtlumenbildenden ersten Katheterschaftelements verschiebbar sein. Die zweite Befestigungsstelle und/oder ein Element des Katheters, an welchem diese vorgesehen ist, etwa das entsprechende Katheterschaftelement, und/oder das verschiebbare Element kann in diesem Beispiel ein Führungselement sein, welches etwa als Gleitelement und/oder Ring oder Schlauchstück ausgebildet sein kann. Das erste Katheterschaftelement kann einen sich distal über das Flüssigkeitslumen heraus erstreckendenSchlauch oder Endabschnitt eines Schlauchs umfassen. Ein solcher Schlauch oder Endabschnitt kann etwa das Drahtlumen bilden und/oder als zumindest teilweise innerhalb eines äußeren Schlauchs aufgenommener innerer Schlauch ausgebildet sein und/oder die Längsachse definieren, entlang welcher die zweite Befestigungsstelle verschiebbar ist. Die zweite Befestigungsstelle kann etwa auf einem solchen Schlauch gelagert und/oder bewegbar sein. Es kann vorgesehen sein, dass die zweite Befestigungsstelle distal eines Flüssigkeitslumens und/oder einer Öffnung eines Flüssigkeitslumens angeordnet und/oder verschiebbar ist, über welche der Ballon gefüllt und/oder entleert werden kann. In entsprechender Weise kann alternativ oder zusätzlich die erste Befestigungsstelle über ein Führungs- oder Gleitelement auf dem ersten Katheterschaftelement in der Längsachse verschiebbar sein.

Generell ist anzumerken, dass ein Ballon auch durch einen Schlauch gebildet sein kann, Bevorzugt ist ein einheitlich durchgängiger Schlauch, der bei Insufflation auf der gesamten Länge gleichmäßig an Durchmesser zunimmt sofern er nicht z.B. durch eine unregelmäßig geformte Arterienwand in der Ausdehnung begrenzt wird. Ein Problem langgestreckter Ballone aus Materialien, die durch geringen Innendruck um ein Vielfaches dehnbar oder hochdehnbar sind, ist die Gleichmäßigkeit der Expansion. Häufig erfolgt die erwünschte Vergrößerung des Ballondurchmessers nur in einem Teil des gesamten Ballons, entweder dem proximalen oder dem distalen Ballonsegment. Beim Füllen kann es zum Bersten des Ballons kommen bevor dieser auf ganzer Länge den gewünschten Durchmesser erreicht hat oder ein weiteres, von dem zuerst insufflierten Segment separiertes Segment nimmt an Durchmesser zu, ohne sich mit dem zuerst gefüllten Segment auf dem gesamten Querschnitt zu verbinden. Zwischen den gefüllten Segmenten verbleibt eine Septe. Eine Septe ist ein Ballonabschnitt, der auch bei hohem Druck eng bleibt. Diese nicht oder sehr wenig aufgedehnten Ballonabschnitte verhindern eine gleichmäßige Behandlung der Gefäßwand über die gesamte Ballonlänge ("sehr wenig": weniger als 30% des Durchmessers senkrecht zur Längsachse der benachbarten, aufgedehnten Ballonsegmente). Septen treten insbesondere dann auf, wenn lange Ballone in Röhren insuffliert werden, die die Ausdehnung senkrecht zur Längsachse begrenzen. Dies ist auch in Blutgefäßen der Fall.

Die Bildung von Septen ist störend und unerwünscht. Sie kann verhindert werden indem die den Ballon bildenden elastischen Schlauchabschnitte vor Gebrauch mindestens einmalig auf der gesamten Länge expandiert werden, ohne daß die Ausdehnung des Ballons senkrecht zur Längsachse eingeschränkt wird. Dies bedeutet eine Ausdehnung des Ballons senkrecht zur Längsachse auf mindestens das 1,1fache, vorzugsweise auf mindestens das 3,0fache, wobei insbesondere bezogen wird auf den Durchmesser des Ballons vor Expansion (Durchmesser = Ausdehnung des Ballons senkrecht zur Längsachse). Aufgrund der elastischen Rückstellkraft erreicht der Ballon nach Verminderung des Innendrucks oder der Beseitigung anderer die Ausdehnung des Ballons verursachender Kräfte wieder annähernd oder vollständig seinen ursprünglichen geringen Durchmesser (vor Expansion). Dieser Vorgang wird als ,Vordehnen' bezeichnet. Zum Vordehnen wird der Ballon bzw. der den Ballon bildende Schlauch z. B. mit Gas soweit gefüllt, daß er auf der gesamten Länge expandiert ist. Die Vordehnung kann einmalig oder mehrmalig für unterschiedlich lange Zeit erfolgen. Durch diese oder auf andere Weise erfolgte Vordehnung der elastischen Membran kommt es bei der späteren Anwendung der Ballone für therapeutische oder diagnostische Zwecke nicht mehr oder nur noch sehr selten zu einer inhomogenen Füllung des Ballons.

Es wurde darüber hinaus überraschend gefunden, daß vorgedehnte und wieder kontrahierte den Ballon bildende Schlauchabschnitte aus dehnbaren Elastomeren sich in Richtung der Längsachse gestreckt auf gebräuchliche Katheterschäfte montieren lassen, ohne daß es selbst bei großen Ballonlängen zu ausgeprägten Verformungen der Katheterschäfte kommt. Die Vorstreckung in der Längsachse bedeutet vorzugsweise, daß ein elastischer Schlauchabschnitt, der ungestreckt bzw. spannungsfrei eine definierte Länge hat, um 20% bis 120% dieser definierten Länge gedehnt und dann unter Beibehaltung dieser Dehnung bzw, unter Spannung auf dem Katheterschaft montiert wird. Beispielsweise wird ein elastischer Schlauchabschnitt, der ungestreckt bzw. spannungsfrei 10 cm lang ist, auf 12 bis 22 cm Länge gedehnt und dann unter Spannung auf dem Katheterschaft montiert. Bevorzugt ist eine Dehnung in der Längsachse um 30 - 80%, besonders bevorzugt eine Dehnung um 40 - 60%. Diese Ballone weisen bei der Insufflation weder Septen noch eine wesentliche weitere Längenausdehnung auf, noch kommt es zu starken Verkrümmungen des unter Druck stehenden Abschnitts des Katheterschaftes. Auf diese Weise ist es möglich besonders lange und im deflatierten Zustand besonders dünne hochelastische Ballonkatheter zu erzeugen.

Die Passage durch ein hämostatisches Ventil kann durch Schutzhüllen erleichtert werden, wobei die Schutzhülle nach Passage des Ballons durch das Ventil entweder auf dem Schaft verbleibt oder abgetrennt wird.

Allgemein können der Ballon und/oder der Katheterschaft an der äußeren Oberfläche physiologisch verträgliche Gleitmittel und / oder Gerinnungshemmer aufweisen. Somit lässt sich der Katheter leicht in eine Schleuse oder einen Führungskatheter einbringen und in einem Blutgefäß verschieben. Das Risiko einer Thrombenauflagerung in Bereichen geringen Blutflusses ist minimiert.

Der Ballon kann mit mindestens einem Arzneimittel beschichtet sein. Somit eignet sich der Ballon zum Einbringen von Arzneimitteln oder anderen Substanzen direkt an behandelte Gefäßwandabschnitte. Der Ballon kann mit einem oder mehreren Arzneistoffen in einer Dosis zwischen 100 µg/cm Ballonlänge und 10 mg/cm Ballonlänge, bevorzugt 300 - 3000 µg/cm Ballonlänge und besonders bevorzugt 600 - 3000 µg/cm beschichtet sein.

Alternativ oder zusätzlich kann der Ballon mit mindestens einem Antioxidans beschichtet sein, bevorzugt mit mindestens einem der folgenden Antioxidantien: Ascorbinsäure, Ascorbylpalmitat, Butylhydroxytoluol, Butylhydroxyanisol, Nordihydroguajaretsäure (NDGA), Propylgallat, Resveratrol.

Die oben genannte Aufgabe wird durch Katheter gelöst, deren Schaft über mindestens zwei Lumina verfügt, wobei eines der Lumina proximal und distal offen für einen Führungsdraht sein kann und als Drahtlumen wirkt und eines als Flüssigkeitszugang zu einem distal montierten elastischen Ballon dienen kann, also als Flüssigkeitslumen. Die distale Öffnung des Flüssigkeitszugangs kann in dem Lumen des elastischen Ballons enden.

Bei den gebräuchlichen aus relativ inelastischem Material bestehenden Ballonen ist die Länge des Ballons durch die Länge des Schlauches auf dem sich der proximale und distale Befestigungspunkt befindet fest begrenzt.

Entsprechend der vorliegenden Erfindung kann darauf verzichtet werden, die Längsausdehnung des Ballons vor Anwendung genau festzulegen. Der Einsatz der Ballone, insbesondere elastischer Ballone, ist nicht auf die Behandlung eines ganz begrenzten Gefäßabschnitt beschränkt. Da die elastischen Ballone anders als die für die Behandlung von Gefäßverengungen eingesetzten gebräuchlichen nicht elastischen Ballone die Gefäßwand mechanisch nicht schädigen sondern sich ihrem Verlauf anpassen, besteht kein Grund den behandelten Bereich proximal oder distal streng zu begrenzen.

Nachfolgend werden Ausführungsformen von Kathetern anhand der beigefügten Figuren erläutert. Es zeigen:
Figur 1 schematisch ein Beispiel für einen Katheter mit dehnbarem Ballon (1), wobei die Längsausdehnung des Ballons durch eine Längsdehnung eines Schlauchs (4) über die gesamte Länge des Schafts bewirkt wird;
Figur 2a schematisch ein Beispiel für einen weiteren Katheter, wobei die Längsausdehnung des Ballons durch eine Verschiebung des distalen Befestigungspunktes (2) auf einem Schlauch (4') mit Endabschnitt (4") bewirkt wird;
Figur 2b schematisch den Katheter der Figur 2a nach Insufflation des Ballons in einem unregelmäßig geformten Lumen mit Ausdehnung des Durchmessers und der Länge des Ballons. A bezeichnet den Bereich des Schlauchs (4') mit dem Endabschnitt (4"), der sich ursprünglich außerhalb des Ballons befand und nach Längenausdehnung des Ballons vom Ballon umgeben ist; und
Figuren 3a und 3b ein Beispiel einer Anwendung eines weichen, in der Form anpassungsfähigen Ballons in einem sich verjüngenden Arteriensegment mit Seitenästen.

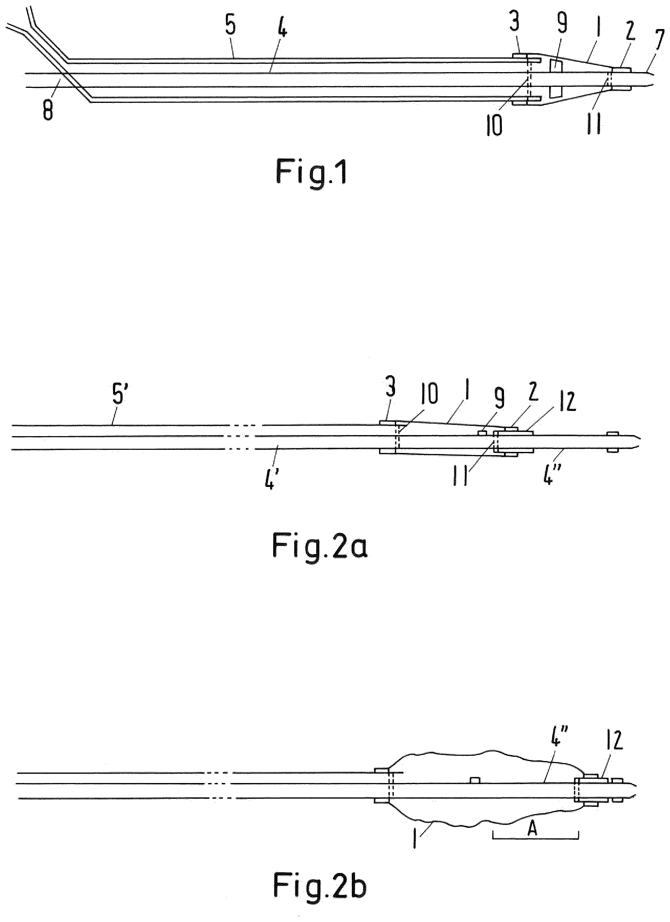

Der Ballon (1), ob in der Längsachse vorgedehnt oder nicht, wird bei Expansion in der Längsausdehnung nicht behindert. Der Ballon kann sich auf einem und gegebenenfalls gemeinsam mit einem zentralen drahtführenden Schlauch in der Längsrichtung ausdehnen, wobei die Verlängerung der Ballonlänge durch Einbeziehung von Schlauchlängen oder Bereichen erfolgt, die sich im ursprünglichen, nicht verlängerten Zustand außerhalb des Ballonlumens bzw. jenseits der Befestigungspunkte des Ballons an dem den Ballon durchlaufenden Schlauch befinden. Das heißt, dem Ballon wird etwa bei Insufflation eine Längenausdehnung dadurch ermöglicht, daß sich der proximale und der distale Befestigungspunkt an den beiden Ballonenden voneinander entfernen, ohne daß das Ausmaß der Entfernung wesentlich durch eine Streckung des relativ kurzen im Ballon befindlichen Schlauchabschnittes bedingt ist. Das Maß dieser möglichen Ausdehnung entlang der Längsachse kann mindestens den Durchmesser des Ballons im insufflierten Zustand betragen, beispielsweise ≥5 mm, bevorzugt ≥ 1cm, stärker bevorzugt ≥ 2cm und am meisten bevorzugt ≥ 4 cm. Dies kann dadurch erreicht werden, daß der Schaft des Katheters aus zwei gegeneinander beweglichen konzentrischen Schläuchen (4, 5) besteht (Figur 1). Der Ballon (1) ist am distalen Ende an einer distalen Befestigungsstelle (2) dicht und fest mit dem inneren Schlauch (4) verbunden, am proximalen Ende an einer proximalen Befestigungsstelle (3) mit dem äußeren Schlauch (5). Somit dient in diesem Beispiel der äußere Schlauch (5) als erstes Katheterschaftelement, an welchem die erste, proximale Befestigungsstelle (3) des Ballons (1) vorgesehen ist, während der innere Schlauch (4) als zweites Katheterschaftelement dient, an welchem die zweite, distale Befestigungsstelle (2) vorgesehen ist. Der innere Schlauch (4) mit geringerem Durchmesser (Lumen bevorzugt 0,035" ~ 0,875 mm oder 0,018"~ 0,45 mm oder 0,014"~ 0,35 mm) weist einen Endabschnitt (4") auf, welcher auch das distale Ende (7) des Katheters bilden kann. Er kann aus elastischem Material bestehen, das sich bei geringem Zug nach distal verlängert. Geringer Zug ist definiert als eine Zugkraft von 1 kg, die distale Verlängerung beträgt bei einer Schaftlänge von 120 cm mindestens 0,5 - 1 cm, bevorzugt ≥ 2 cm, stärker bevorzugt ≥ 3 cm und am meisten bevorzugt ≥ 4 cm. Da die Länge des inneren Schlauches (4) wesentlich länger ist als die des Ballons, erlaubt eine geringe prozentuale Dehnung des Schlauches über seine Länge eine bedeutende Verlängerung der Ballons. Um ein Verschieben des inneren Schlauches (4) in proximale Richtung unter Verkürzung des Ballons z.B. beim Einführen des Katheters durch eine Schleuse zu verhindern, kann der innere Schlauch (4) mit einem Stopper oder Anschlag (9) versehen werden, der wegen seines größeren Durchmessers ein Zurückrücken des inneren Schlauches (4) in den äußeren Schlauch (5) verhindert. Alternativ zur Dehnbarkeit in Richtung der Längsachse oder zusätzlich kann der innere Schlauch (4) gegenüber dem äußeren Schlauch (5) auf der gesamten Länge in Längsrichtung verschiebbar sein; in diesem Falle kann eine die Bewegung erlaubende Dichtung optional mit Feststeller (8) vorhanden sein. Damit ist der innere Schlauch (4) durch den Arzt manuell verschiebbar und/oder er wird durch den beim Füllen des Ballons entstehenden Druck entsprechend der Längsausdehnung des Ballons verschoben. Der Ballon kann durch den als Flüssigkeitslumen dienenden Zwischenraum zwischen Schlauch (5) und (4) gefüllt und entleert werden.

Der Ballon besteht aus natürlichen oder synthetischen polymeren Elastomeren, bevorzugt aus Gummi, Latex, Polyrurethan, Isopren-basierten Polymeren oder Copolymeren, Silikon, besonders bevorzugt aus Latex, Polyurethan oder ChronoPrene^{®} von Cardio Tech International, Inc., jetzt AdvanSource Biomaterials, Inc., USA, ohne oder mit Zusätzen oder aus Gemischen verschiedener Bestandteile. Weitere geeignete Polymere sind in den bereits genannten Patentschriften (US 20090258049, US 8066667, US 8216267, US 20080243103, US 7179251, WO2012158944) zu finden. Andere Elastomere mit der Dehnfähigkeit der oben genannten Produkte und Polymere sind ebenfalls geeignet. Als Zusätze sind Substanzen zur Verbesserung der mechanischen Eigenschaften, der Dauer der Haltbarkeit oder auch der Kontrastdichte von besonderem Interesse. Die Kontrastdichte der Ballonmembran läßt sich durch den Zusatz jodierter Kontrastmittel oder z.B. durch Metallpulver steuern.

Von besonderem Interesse sind Katheter für die Behandlung langgestreckter peripherer Arterien, deren Durchmesser sich über die Länge deutlich vermindert. Die Länge der insufflierten oder ausgedehnten Ballone kann 2 cm bis 40 cm oder mehr betragen, bevorzugt sind Längen zwischen 5 und 35cm, mehr bevorzugt Längen zwischen 10 und 30 cm, besonders bevorzugt Längen zwischen 20 und 30 cm. Die Länge der Ballone vor Insufflation oder Ausdehnung kann ≥ 10 cm, bevorzugt ≥ 15 cm und besonders bevorzugt ≥ 20 cm betragen.

Die Füllung und insbesondere die Entleerung derartig großvolumiger Ballone mit Kontrastmittel erfordert Zeit. Alternativ ist eine Befüllung mit absorbierbaren Gasen, z.B. Kohlendioxid möglich.

Der äußere Durchmesser eines Ballons kann zwischen 0,5 und 2,5 mm liegen, bevorzugt zwischen 1 und 2 mm. Das Ballonmaterial kann diese engen Durchmesser entweder und bevorzugt im nicht expandierten Zustand aufweisen oder es kann um den Schaft gefaltet und optional mittels eines zurückziehbaren dünnwandigen Schlauches oder einer vergleichbaren Vorrichtung fixiert werden.

Ballone für kleine Gefäße wie Koronararterien weisen im nicht expandierten Zustand einen Durchmesser von 0,5-1,5 mm auf, im expandierten Zustand bis zu 6 mm. Die bevorzugten Längen sind den Längen der zu behandelnden Gefäße angepaßt, d.h. für Koronargefäße mindestens das 10fache des mittleren Durchmessers des zu behandelnden Segments, beispielsweise bei einem mittleren Gefäßdurchmesser von 3 mm beträgt die Ballonlänge erfindungsgemäß alternativ ≥30 mm,

Die Ballonenden werden etwa mittels Kleber oder durch Schweißen/ Hitze oder mit Lösungsmitteln durch Anlösen oder mittels Fäden oder mit einer Kombination der verschiedenen Verfahren an den Befestigungsstellen befestigt, etwa mit dem Schaft oder Schlauch oder einem auf dem Schlauch verschiebbarem Führungselement wie einem Rohr oder Ring oder Schlauchstück verbunden. Der Ballon kann an mindestens 2 Stellen direkt oder indirekt über ein Gleitelement an einem zentralen, den Ballon in Längsrichtung durchquerenden Schlauch befestigt sein, insbesondere an einem ein Drahtlumen definierenden Schlauch und/oder inneren Schlauch. Mehr Befestigungspunkte sind möglich. Die Befestigung kann an den unterschiedlichen Befestigungspunkten auf unterschiedliche Art erfolgen.

Eine Möglichkeit eine Längenveränderung des Ballons bei Aufrechterhaltung der prinzipiell zylindrischen Form zuzulassen besteht darin, zumindest einen der Befestigungspunkte des Ballons auf der Längsachse verschiebbar zu gestalten (Figur 2). Als konstante Achse wird der den Ballon passierende engere Schlauch (4') gewählt, der als Drahtführungsschlauch dient. Dieser ist etwa bis zur ersten, proximalen Befestigungsstelle (3) in einem Doppellumenschlauch geführt, welcher einen parallel zum Drahtführungsschlauch (4') geführten Schlauch (5') aufweist, der ein Flüssigkeitslumenbildet. Der Drahtführungsschlauch (4') weist einen Endabschnitt (4") auf, welcher sich distal über den das Flüssigkeitslumen bildenden Schlauch (5') hinaus erstreckt. Durch den Schlauch (4') und den Endabschnitt (4") wird eine Längsachse definiert. Auf denEndabschnitt (4")wird beispielsweise einige Zentimeter vom distalen Ende entfernt als Führungselement ein gleitfähiges, jedoch eng anliegendes Schlauchstück(12) oder ein entsprechendes gleitfähiges, eng anliegendes kurzes Rohr positioniert. Das Führungselement ist in diesem Beispiel somit als Gleitelement ausgebildet. Das distale Ballonende (2) wird an dem beweglichen Schlauchstück (12) abdichtend befestigt. In diesem Beispiel kann der Doppellumenschlauch beziehungsweise der Drahtführungsschlauch (4') und/oder der relativ zu diesem fixierte Schlauch (5') als erstes Katheterschaftelement angesehen werden, an welchem die erste, proximale Befestigungsstelle (3) zur direkten oder indirekten Befestigung des Ballons vorgesehen ist. Am relativ dazu verschiebbaren Schlauchstück (12) ist die zweite, distale Befestigungsstelle (2) des Ballons vorgesehen. Bei Expansion des Ballons kann das bewegliche Schlauchstück (12) auf dem festen Schlauch (4'), insbesondere dem Endabschnitt (4"), seine Position so ändern, daß sich der Ballon in der Längsrichtung ausdehnen kann. Das Schlauchstück (12) kann im Inneren des Ballons so lang gewählt werden, daß es die Ballonlänge auf einen festgelegten Minimalwert fixiert. Die Verschiebung des Schlauchstücks (12) in proximale Richtung wird beispielsweise durch den Schlauch (5') begrenzt oder durch andere Sperren oder Anschläge (9) innerhalb des Ballonlumens. Eine Verschiebung des als Gleitelement ausgebildeten Schlauchstücks (12) nach distal über das distale Ende des Schlauches (4') oder des Endabschnitts (4") kann durch einen entsprechenden Anschlag distal oder durch eine die Bewegung nach distal begrenzende Befestigung an dem Schlauch (5') erreicht werden. Das Schlauchstück (12) muß gegenüber dem Schlauch (4') nicht vollständig abdichten, da der Druck im Lumen des Ballons nur gering höher zu sein braucht als der Blutdruck und bei länger andauernder Ballonexpansion Druck und Volumen über die das Ballonlumen befüllende Spritze nachreguliert werden können. Allgemein kann das Führungselement als Ring und/oder Rohr und/oder Schlauchstück ausgebildet sein, insbesondere als kurzes Metallrohr ausgebildet sein und/oder als röntgendichter Schlauch. Alternativ oder zusätzlich kann das Führungselement allgemein insbesondere im Bereich des Ballonansatzes einen Röntgenmarker (11) aufweisen.

Da die Längsbewegung des Schlauches in diesem Fall nicht über eine elastische Verlängerung des Schlauches (4') erfolgen muß, kann der Katheterschaft allgemein etwa ein einheitlicher Doppellumenschlauch sein. Eines der Lumina, insbesondere das durch den Schlauch (4') gebildete Drahtlumen, setzt sich durch den Ballon nach distal fort, das andere Lumen füllt oder entleert den Ballon und wirkt als Flüssigkeitslumen.

Die oben beschriebenen Maßnahmen verhindern oder begrenzen eine unregelmäßige Verformung eines sich in Längsrichtung unter Druck ausdehnenden, an 2 Enden fixierten Ballons. Dies betrifft insbesondere Ballone, die bei einem Druck von ≤ 4 bar und bei freier Ausdehnung mehr als 5% ihrer ursprünglichen (druckfrei gemessenen) Länge zunehmen. Bevorzugt eingesetzt werden die oben beschriebenen Verfahren für Ballone, die unter diesen Bedingungen ≥ 10% an Länge zunehmen, mehr bevorzugt für Ballone, die ≥ 25% an Länge zunehmen. Die Länge des Ballons wird entsprechend der vorliegenden Erfindung nicht durch innere oder äußere begrenzende Elemente wie z.B. einen zurückziehbaren äußeren Schlauch begrenzt.

Im Übrigen entspricht der Katheteraufbau demjenigen gebräuchlicher Angioplastiekatheter in Bezug auf Materialien, Maße, den Griff, Art und Anzahl der Ansätze für Spritzen, dem Katheterschaft, mit röntgendichten oder magnetischen Markern (10, 11) bevorzugt nahe der Fixierungspunkte des Ballons am Schaft, mit dem Lumen zum Befüllen und Entleeren des Ballons und mit einem Lumen für einen Führungsdraht, entweder über die gesamte Schaftlänge oder nur den distalen Teil desselben (rapid-exchange Version). Der Schaft kann mit mehr als einem Ballon versehen sein und mit mehr Lumina als einem Draht- und einem Flüssigkeitslumen ausgestattet sein. Der Schaft oder Teile desselben und der Ballon können durch spezielle Beschichtungen gleitfähig gemacht werden.

Der elastische Ballon kann mit einem oder mehreren Gleitmittel oder mit einem oder mehreren Arzneimitteln oder mit beliebigen Gemischen von Gleit- und Arzneimitteln beschichtet sein. Der Beschichtung können Hilfsstoffe oder Zusätze zugesetzt werden. Wird die Beschichtung in flüssiger Form aufgetragen, eignen sich Lösungsmittel, die das Ballonmaterial nicht oder wenig angreifen und die sich gleichmäßig auf der Ballonoberfläche verteilen. Bevorzugt sind leicht flüchtige Lösungsmittel wie beispielsweise Methanol, Ethanol, Propanol oder Isopropanol, Aceton, Dimethylformamid, Acetonitril, Ethylacetat, Hexan, Heptan oder Gemische derselben und als weiteres Lösungsmittel oder als Zusatz zu organischen Lösungsmitteln Wasser.

Geeignete Beschichtungslösungen, -zusammensetzungen und -verfahren sind u.a. in oben genannten Patentschriften beschrieben. Die elastischen Ballone können im kontrahierten oder gering oder voll expandierten Zustand beschichtet werden. Um eine gleichmäßige teilweise Expansion zu erzielen werden die Ballone in einem ggf. mit einer Gleitschicht versehenen Rohr expandiert, der Innendruck des Ballons wir aufrecht erhalten oder geringfügig vermindert, die Ballone werden aus dem Rohr entnommen und dann in üblicher Weise mit der flüssigen Formulierung beschichtet. Nach dem Beschichten vor oder während oder nach dem Abdampfen des oder der Lösungsmittel werden die Ballone evakuiert, so daß sie entweder durch Kontraktion der elastischen Membran den gewünschten kleinen Querschnitt erreichen oder sie werden gefaltet und die Falten um den Schaft gerollt wie das z.B. bei Ballonen aus Nylon üblich ist.

Bevorzugte Wirkstoffe sind Paclitaxel und andere Taxane, Rapamycin und andere TORbindende Wirkstoffe wie Everolimus, Zotarolimus, Biolimus etc., Kortikoide und deren lipophile Derivate wie Betamethasondiproprionat oder Dexamethason-21-palmitat, Statine wie z.B. Atorvastatin, Cerivastatin oder Fluvastatin; Gerinnungshemmer und Plättchenaggregationshemmer wie Heparin und dessen Fragmente oder Derivate, Hirudin, Acetylsalicylsäure, Ticlopidin, Clopidogrel, Prasugrel, Iloprost oder andere Prostacycline oder Prostaglandine wie E1, E2, F2α oder Warfarin, Phenprocoumon, besonders bevorzugt sind Paclitaxel, Rapamycin und Betametasondipropionat einerseits und Gerinnungshemmer wie Heparin und Hirudin andererseits. Die Wirkstoffe bzw. deren Salze können einzeln oder in Kombination miteinander eingesetzt werden. Geeignete Dosierungen können zwischen 100 µg/cm Ballonlänge und 10 mg/cm Ballonlänge liegen, bevorzugt sind 300 - 3000 µg/cm Ballonlänge und besonders bevorzugt 600 - 3000 µg/cm. Wird ein Ballon mit mehr als einem Wirkstoff beschichtet beziehen sich die obigen Zahlen auf die Summe der Dosierungen der einzelnen Wirkstoffe. Weiterhin kann der Ballon an der Oberfläche oder in einer Struktur der Ballonmembran allein oder in Kombination mit synthetischen Wirkstoffen biologische Substanzen wie zum Beispiel Eiweiße, Polysaccaride, Pentagalloylglucose, Lipide wie Nitroölsäure, Hormone und/ oder Nukleinsäuren enthalten; besonders geeignet sind Gelatine und andere, auch synthetische Gelbildner, Antikörper, mikro RNA, microRNA-Inhibitoren wie Inhibitoren von miR-29, bevorzugt ist Gelatine, besonders bevorzugt ist Nitroölsäure. Somit eignet sich der Ballon zum Einbringen von Arzneimitteln oder anderen Substanzen direkt an behandelten Gefäßwandabschnitten.

Überraschend ist die hohe Beladbarkeit trotz geringer verfügbarer Oberfläche der Ballone im kontrahierten Zustand. Bei den gebräuchlichen wenig elastischen Nylon-Ballonen steht die gesamte endgültige Fläche der Ballonmembran für die Beschichtung zur Verfügung, z.B. bei einem Ballon von 6 mm Durchmesser und 5 cm Länge eine (zylindrische) Fläche von 942mm². Der geringe Querschnitt der Nylon-Ballone für die Einführung in die Arterien wird durch Falten erreicht, wodurch sich die Membranoberfläche nicht ändert.

Ein Chronoprene^{™} Ballon für ein Gefäß mit gleichem Durchmesser (6 mm) hat aber in kontrahiertem Zustand für die Beschichtung nur eine Fläche von Abmessungvon 235mm² (äußerer Durchmesser kontrahiert 1,5mm) oder 314mm² (äußerer Durchmesser kontrahiert 2,0mm), trägt aber die gleiche Arzneistoffdosis. Dies bedeutet bei der gebräuchlichen Dosis von 3µg/mm² bei einem Nylonballon 12 bzw. 9 µg/mm² bei einem Chronoprene Ballon im Zustand bei Beschichtung bzw. beim Einführen in eine Arterie. Ausreichend für die Beschichtung elastischer oder hochelastischer Ballone im kontrahierten Zustand ist eine Beschichtung mit ≥5µg/ mm², bevorzugt wird eine Beschichtung mit >10µg/mm² der Oberfläche.

Wegen der leichten Verformbarkeit der elastischen Membranen kann es wichtig sein, die Reibung der Beschichtung an Ventilen, Einführschleusen und Führungskathetern und in den Blutgefäßen gering zu halten. Die Verminderung der Reibung ist umso wichtiger, je länger der Ballon ist. Die Reibung dehnbarer Ballons kann zu einer Verschiebung des Ballonmaterials in der Längsachse, zu Falten und Verformungen, die unerwünscht sind und zu Verlusten an Arzneistoff sowie zu einer ungleichmäßigen Übertragung des Arzneistoffs auf die Gefäßwand führen. Zur Verbesserung der Gleitfähigkeit können physiologisch verträgliche, bevorzugt feste Gleitmittel verwendet werden, beispielsweise Magnesiumstearat, Calciumstearate, Zinkstearate, Magnesiumpalmitat, Calciumpalmitat, Zinkpalmitat, Magnesiummyristat, Calciummyristat, Magnesiumlaurat, Calciumlaurat, Magnesiumcaprinat, Calciumcaprinat, Magnesiumcaprylat, Calciumcaprylat, Magnesiumoleat, Calciumoleat, Magnesiumpalmitoleat oder Calciumpalmitoleat. Eine oder mehrere der folgenden Substanzen können zugesetzt werden: Stearinsäure, Palmitinsäure, Laurinsäure, Caprinsäure, Caprylsäure, Ölsäure, Palmitoleinsäure, Stearylalkohol, Palmitylalkohol, Laurylalkohol, Magnesiumacetat und/ oder Calciumacetat, sowie organische Salze der genannten Fettsäuren wie z.B. Salze mit Ethanolamin, Aminopropandiol, Serinol, Glucosamin, Tris (Tris(hydroxymethyl)aminomethan), Methylglucamin, basischen Aminosäuren wie Lysin oder Arginin. Bevorzugt sind saure, neutrale oder allenfalls schwach basische Fettsäuresalze oder Gemische. Diese Substanzen können einzeln oder in Mischung miteinander oder in Verbindung mit einem oder mehreren pharmakologisch wirksamen Substanzen verwendet werden. Beschichtungen, die die Gleitfähigkeit verbessern, können auf die Ballonmembran, aber auch auf den Katheterschaft aufgetragen werden.

Weiterhin können Zusätze und pharmazeutische Hilfsstoffe zur chemischen oder pharmazeutischen Stabilisierung der Beschichtung oder auch der Ballonmembran zugesetzt werden. Bevorzugt werden Antioxidantien wie beispielsweise Ascorbinsäure, Ascorbylpalmitat, Butylhydroxytoluol, Butylhydroxyanisol, Nordihydroguajaretsäure, Propylgallat, Resveratrol, besonders bevorzugt Nordihydroguajaretsäure, Propylgallat, Resveratrol. Geeignet sind weiterhin Substanzen, die sich bei der Beschichtung der gebräuchlichen Angioplastie-Ballone bewährt haben, insbesondere Röntgenkontrastmittel und Harnstoff. Zahlreiche mögliche Zusatzstoffe wurden in (EP 2170421, US 8244344) beschrieben.

Auch Ascorbylpalmitat ist wegen seiner vielfältigen Eigenschaften von besonderem Interesse.

Werden die Ballone mit mehreren Komponenten beschichtet, kann die Beschichtung mit allen Komponenten gleichzeitig erfolgen oder zunächst mit einer der Komponenten oder einem der Komponentengemische und danach mit einer oder mehreren weiteren Komponenten. Substanzen, die die Gleitfähigkeit des Ballons erhöhen, können bevorzugt als äußere Deckschicht aufgetragen werden.

Die Beschichtung kann mit gebräuchlichen Verfahren im kontrahierten, teils oder ganz expandierten Zustand mit einer flüssigen Zubereitung durch Tauchen, Sprühen oder das Auftragen eines definierten Volumens erfolgen oder auch durch Drucktechnik oder mit Hilfe von Feststoffen, beispielsweise als Pulver oder mit in der Größe definierten Mikro- und/ oder Nanopartikeln.

Ein mögliches Herstellungsverfahren von Ballonkathetern mit beschichteten dehnbaren Ballonen kann allgemein wie folgt beschrieben werden:
1. Bereitstellung von Schläuchen (=Ballonen) aus elastischen Materialien wie Gummi, Latex, Polyrurethan, Isopren-basierten Polymeren oder Copolymeren, Silikon, besonders bevorzugt aus Latex oder ChronoPrene^{®}
2. Vordehnen der elastischen Schläuche senkrecht zur Längsachse über die gesamte Länge, bevorzugt um das 3fache oder mehr des ursprünglichen Durchmessers beispielsweise durch Insufflation, Rückführung in den ursprünglichen Zustand durch Druckminderung, d.h. auf den ursprünglichen Durchmesser
3. Bereitstellung von koaxialen Katheterschäften mit einem inneren Schlauch mit Drahtlumen, einem Flüssigkeitslumen, einem Ansatz für eine flüssigkeitsgefüllte Spritze und röntgensichtbaren Markern am proximalen und distalen Befestigungspunkt des elastischen Schlauches
4. Platzierung des distalen Abschnitts des Katheterschaftes in dem Schlauch, so daß der distale Auslaß des Flüssigkeitslumens des Katheterschaftes in dem Schlauch zu liegen kommt und der proximale röntgensichtbare Marker distal des proximalen Schlauchendes liegt, z.B. etwa 3 mm distal des proximalen Schlauchendes. Das Drahtlumen soll etwas mehr als eine Schlauchlänge distal aus dem elastischen Schlauch herausragen.
5. Befestigung des proximalen Schlauchendes am Schaft durch Kleben und Sicherung mittels eines chirurgischen Fadens.
6. Strecken des elastischen Schlauches in der Längsrichtung auf das 1,2 bis 2,2fache der ursprünglichen Länge
7. Befestigen des Schlauches ca. 1 cm proximal des distalen das Drahtlumen enthaltenden Schlauchendes durch Kleben und Sicherung mittels eines chirurgischen Fadens.
8. Visuelle Prüfung
9. Prüfung auf Dichtigkeit durch Anlegen von Vakuum und/ oder Plazierung in einer Röhre und Anlegen von Druck von 2,0266-4,0532 bar (2-4 atm) Deflation
10. Verpacken und Sterilisieren

Die folgenden Beispiele zeigen den Bau der Ballone, die Beschichtung mit einem ausgewählten Arzneistoff, dessen Haftung am Ballonmaterial, die Funktionsweise bei Expansion in Blutgefäßen sowie die Freisetzung des Wirkstoffs bei Expansion im Gewebe und eine für die Wirkung gut ausreichende Übertragung in die Gefäßwand.

### Beispiel 1

Polyurethan-Ballone, die im deflatierten Zustand 2mm Durchmesser aufweisen und 5 cm lang sind, am distalen Ende von Kathetern montiert, wurden mit 40 µl einer Lösung von Paclitaxel in Aceton/Wasser, 50mg/ml mittels einer Hamilton Mikrospritze beschichtet. Auf den Ballonen fanden sich bei Analyse 1707 µg Paclitaxel. Wurden die Ballone in trockenem Zustand auf einen Durchmesser von 10 mm expandiert, verblieben noch immer 80 % des Paclitaxel auf der Ballonoberfläche. Nach Expansion mit ca. 0,5 bar in der Arteria iliaca eines Schweins wurden 15% der Dosis in dem Arteriengewebe gefunden, 6% verblieben auf dem Ballon.

### Beispiel 2

Kleine Ballone mit Silikonmembran, die im deflatierten Zustand 2mm Durchmesser aufweisen, 1 cm lang sind, am distalen Ende von Kathetern montiert, wurden mit einer Lösung von Paclitaxel (50 mg/ml) und Nordihydroguajaretsäure (10 mg/ml) in Aceton/Ethanol/Wasser beschichtet. Auf den Ballonen fanden sich bei Analyse 637 µg Paclitaxel. Wurden die Ballone in trockenem Zustand mit 2 bar auf einen Durchmesser von 10 mm expandiert, verblieben noch immer 87 % des Paclitaxel auf der Ballonoberfläche.

### Beispiel 3

Latex-Ballone, die nach Vorstrecken 5 cm lang sind, wurden auf Katheterschäften mit einem Drahtlumen von 0,0889 cm (0,035") Durchmesser montiert. Die Ballone wurden mit 40 µl einer Lösung von Paclitaxel (50 mg/ml) und Nordihydroguarajetsäure (15mg/ml) in Aceton/ Ethanol/ Wasser mittels einer Hamilton Mikrospritze beschichtet. Auf den Ballonen wurden 1752±30 µg Paclitaxel gefunden. Die Ballone wurden mit einem maximalen Druck von 2 bar in peripheren Arterien von Schweinen insuffliert. Kurze Zeit später wurden in den Arterienwänden von Schweinen 4,4±2,5 % der Dosis gefunden, 9±9 % der Dosis waren auf den Ballonen nach Entnahme aus den Tieren verblieben.

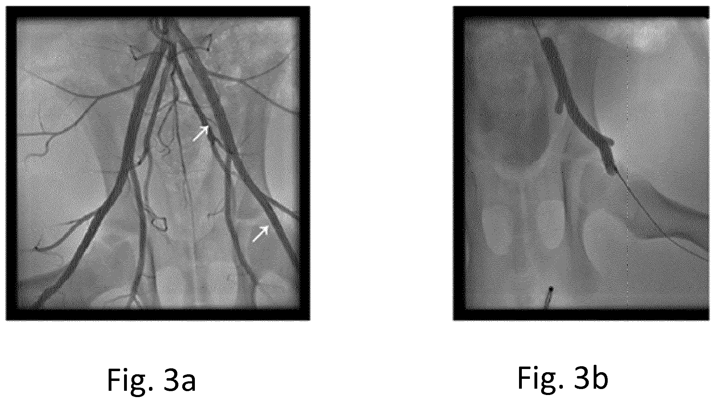

Die Röntgenaufnahmen der Figuren 3a und 3b zeigen in Figur 3b die Anpassung eines Ballons an den nach distal abnehmenden Durchmesser und die Seitenäste der behandelten Arterie. In Figur 3a ist der behandelte Bereich durch Pfeile markiert. Die Behandlung führt zu einer Glättung der Gefäßwände, dem Anlegen von Dissektionen an die Wand und gegebenenfalls dem Übertritt eines Arzneistoffs ins Gewebe.

### Beispiel 4

Chronopren^{™} -Ballone von 2,5 cm Länge und 2 mm Außendurchmesser wurden auf 4,5 cm Länge vorgedehnt und auf Katheterschäften mit einem Drahtlumen von 0,0889 cm (0,035") Durchmesser montiert. Die Ballone wurden mit 50 µl einer Paclitaxellösung mit 50 mg/ml in 75% Aceton/ 25% Methanol beschichtet. Der Arzneistoffgehalt der Ballone nach Beschichtung betrug 2104±248µg. Nach Passage durch ein hämostatisches Ventil, einen 1m langen mit Blut gefüllten Führungskatheter und 1 min Aufenthalt in bewegtem Blut fanden sich noch 81±8% der Dosis auf den Ballonen; kurze Zeit nach Inflation der Ballone für 1 min in einer peripheren Arterie des Schweins mit ca. 1 bar wurden 6% der Dosis im Gewebe gefunden. Dies entspricht weitgehend der Übertragung von Wirkstoff durch nicht dehnbare Ballone bei Anwendung hoher Drücke (Scheller et al. 2004).

### Beispiel 5

Chronoprene^{™} -Ballone, Dunn Industries Inc, USA, 2 mm äußerer Durchmesser, wurden vor der Verarbeitung ohne äußere Begrenzung an der Luft bei ca. 1 bar vollständig expandiert (Durchmesser über die gesamte Länge ca. 10 mm), deflatiert (zurück auf den ursprünglichen Durchmesser von 2 mm). Segmente mit 10 cm Länge wurden auf 20 cm Länge vorgestreckt auf Koaxialschäften mit 0,0889 cm (0,035") Drahtlumen entsprechend Fig 1 montiert und in Bezug auf Dichtigkeit geprüft.. Die Katheter wurden in gebräuchlicher Weise mit Ethylenoxid sterilisiert. Nach Einführen in die Beinarterien von Schweinen wurden die Ballone mit 4-7 ml Kontrastmittel gefüllt. Der Druck im Katheterlumen betrug < 0,50665 bar (0,5atm). Die Ballone waren trotz des geringen Druckes gleichmäßig expandiert, der Ballondurchmesser entsprach dem Gefäßverlauf, proximal ca. 7 mm, distal 4 mm, mit erkennbarer Füllung der Abgänge von Seitenästen ähnlich Fig. 3b.

### Beispiel 6

Chronoprene^{™} -Ballone, Dunn Industries Inc, USA, 1,5 mm äußerer Durchmesser Innendurchmesser 1,1 mm, 18 mm auf 36 mm vorgestreckt auf Rx Schäften 0,0356 cm (0,014") montiert, im nicht-inflatierten Zustand beschichtet mit 25 mg/mL Paclitaxel +1,25 mg/mL Nitroölsäure+ 5 mg/mL NDGA in Aceton/MeOH/H₂O, Auftragsvolumen = 54 µl; Paclitaxelgehalt der Ballone: 1140±69µg (=Dosis); nach Passage durch ein hämostatisches Ventil, einen 1m langen mit Blut gefüllten Führungskatheter und 1 min Aufenthalt in bewegtem Blut fanden sich noch 87±11% der Dosis auf den Ballonen.

Die Untersuchung zeigt eine gute Haftung des Wirkstoffs auf dem Ballon bei der Simulation des Wegs zur Arterie, die behandelt werden soll.

### Beispiel 7

Chronoprene^{™} -Ballone, Dunn Industries Inc, USA, 1,5 mm äußerer Durchmesser Innendurchmesser 1,1 mm, Q-Medics Schaft biluminal 0,046 cm (0,018"), 20 mm auf 40 mm vorgestreckt, im nicht-inflatierten Zustand beschichtet mit 25 mg/mL Paclitaxel +1,25 mg/mL Nitroölsäure in Aceton/MeOH/H₂O Auftragsvolumen = 54 µl; Paclitaxelgehalt der Ballone: 1236±27 µg (=Dosis); die Ballone wurden in die A. iliaca interna oder externa von Schweinen eingeführt, dort für 1 min expandiert, deflatiert und zurückgezogen. Die Tiere wurden 10 min nach Behandlung in Narkose getötet und der Paclitaxelgehalt auf den gebrauchten Ballonen sowie in den behandelten Arterienwänden mittels HPLC/ UV-Detektion bestimmt. Es verblieben 5,8±3,3 % der Dosis auf den Ballonen, 14,0±5,0% der Dosis wurden in den Gefäßwänden gefunden (jeweils n=6).

Die Untersuchung zeigt die weitgehend vollständige Abgabe des Arzneistoffs vom Ballon und die Übertragung eines nach bisherigen Kenntnissen wirksamen Anteils der Dosis auf die Gefäßwand.

### Bezugszeichenliste

- 1: Ballonmembran
- 2: distale Befestigungsstelle des Ballons
- 3: proximale Befestigungsstelle des Ballons
- 4: Katheterschaft/innerer Schlauch mit Drahtlumen
- 5: Katheterschaft/äußerer Schlauch mit Flüssigkeitslumen
- 7: distales Ende des Katheters
- 8: Abdichtung
- 9: Anschlag
- 10: Röntgenmarker
- 11: Röntgenmarker
- 12: Schlauchstück
- 4': Katheterschaft, Drahtlumen
- 4": Endabschnitt
- 5': Katheterschaft einschließlich Flüssigkeitslumen

## Patentansprüche

1. Ballonkatheter mit:
einem Ballon (1), wobei der Ballon (1) eine Ballonmembran aufweist, die aus einem oder mehreren dehnbaren Elastomeren besteht; einem gebräuchlichen Katheterschaft, welcher ein Drahtlumen bildet, das zum Führen eines Führungsdrahtes ausgebildet ist, und ein Flüssigkeitslumen bildet, über welches dem Ballon (1) eine Flüssigkeit zuführbar ist;
wobei der Ballon (1) an einer ersten, proximalen Befestigungsstelle (3) an einem ersten Katheterschaftelement (5, 4', 5') befestigt ist; und
der Ballon (1) an einer zweiten, distalen Befestigungsstelle (2) an einem zweiten Katheterschaftelement (4, 12) befestigt ist, wobei ferner der Ballon (1) einen Abschnitt des Katheterschafts umgibt, welcher sich zwischen der ersten und zweiten Befestigungsstelle (3, 2) erstreckt;
und der vom Ballon (1) umgebene Abschnitt des Katheterschafts mit sich ändernder Längenausdehnung des Ballons (1) unterschiedliche Bereiche des Katheterschafts umfasst;_**dadurch gekennzeichnet,**
**dass** die Länge des Ballons (1) im nicht ausgedehnten Zustand ≥ 10 cm oder bei einem Durchmesser von 3 mm ≥ 30 mm beträgt.

2. Ballonkatheter nach Anspruch 1, wobei die zweite Befestigungsstelle (2) gegenüber der ersten Befestigungsstelle (3) entlang einer Längsachse des Katheterschafts bewegbar ist.

3. Ballonkatheter nach Anspruch 1 oder 2, wobei das eine oder die mehreren dehnbaren Elastomeren ausgewählt sind aus Gummi, Latex, Polyurethan, Isopren-basierten Polymeren oder Copolymeren, Silikon, besonders bevorzugt aus Latex, Polyurethan oder einem Copolymer aus Polystyrol und hydriertem Isopren.

4. Ballonkatheter nach einem der Ansprüche 1-3, wobei die Länge des Ballons (1) im nicht ausgedehnten Zustand bevorzugt ≥ 15 cm und besonders bevorzugt ≥ 20 cm beträgt.

5. Ballonkatheter nach einem der Ansprüche 2-4, wobei das zweite Katheterschaftelement (12) als auf dem ersten Katheterschaftelement (4', 5') gelagertes Führungselement ausgebildet ist, welches entlang einer Längsachse des das Drahtlumen bildenden ersten Katheterschaftelements (4', 5') auf dem ersten Katheterschaftelement (4', 5') verschiebbar ist.

6. Ballonkatheter nach einem der Ansprüche 1 bis 5, wobei der Ballon bei einem Innendruck von < 2,0266 bar (2 atm) über dem Außendruck seinen Durchmesser mindestens auf das 2,5 fache vergrößert, der Ballon in der Längsachse um mindestens 20% gegenüber seinem Ruhezustand gestreckt ist und nach der Befestigung auf dem ersten und zweiten Katheterschaftelement in der Längsachse unter Spannung verbleibt, **dadurch gekennzeichnet, daß**
das elastische Ballonmaterial senkrecht zur Längsachse um mindestens den Faktor 3 vorgedehnt wurde und in dieser Richtung spannungsfrei ist.

7. Ballonkatheter nach einem der Ansprüche 1 bis 6, wobei der Ballon (1) und/oder der Katheterschaft an der äußeren Oberfläche physiologisch verträgliche Gleitmittel aufweist.

8. Ballonkatheter nach einem der Ansprüche 1 bis 7, wobei der Ballon (1) mit mindestens einem Arzneimittel beschichtet ist, bevorzugt mit mindestens einem der folgenden Arzneimittel: Paclitaxel, anderen Taxanen, Rapamycin, Everolimus, Zotarolimus, Biolimus, Betamethasondiproprionat, Dexamethason-21-palmitat, Atorvastatin, Cerivastatin, Fluvastatin, Heparin, Hirudin, Acetylsalicylsäure, Ticlopidin, Clopidogrel, Prasugrel, Iloprost, Prostaglandine E1, E2, F2α, Warfarin, Phenprocoumon.

9. Ballonkatheter nach Anspruch 8, wobei der Ballon (1) mit einem oder mehreren Arzneistoffen in einer Dosis zwischen 100 µg/cm Ballonlänge und 10 mg/cm Ballonlänge, bevorzugt 300 -3000 µg/cm Ballonlänge und besonders bevorzugt 600 - 3000 µg/cm beschichtet ist.

10. Ballonkatheter nach einem der Ansprüche 1 bis 9 wobei der Ballon (1) mit mindestens einem Antioxidans beschichtet ist, bevorzugt mit mindestens einem der folgenden Antioxidantien: Ascorbinsäure, Ascorbylpalmitat, Butylhydroxytoluol, Butylhydroxyanisol, Nordihydroguajaretsäure, Propylgallat, Resveratrol, und/ oder Nitroölsäure.

11. Ballonkatheter nach Anspruch 1 bis 10, wobei der Ballon (1) vor Gebrauch vorbehandelt wird, **dadurch gekennzeichnet, dass** der Ballon (1) mindestens einmalig auf der gesamten Länge senkrecht zur Längsachse auf mindestens das 1,1fache, bezogen auf den Durchmesser des Ballons (1) vor Expansion, expandiert wird, ohne dass die Ausdehnung des Ballons (1) senkrecht zur Längsachse eingeschränkt wird.

12. Ballonkatheter nach Anspruch 1 bis 11, **dadurch gekennzeichnet, dass** zur Montage des Ballons (1) auf einem gebräuchlichen Katheterschaft der Ballon (1) in der Längsachse um 20% bis 120% gegenüber seinem Ruhezustand gedehnt wird und unter Beibehaltung dieser Dehnung auf dem Katheterschaft montiert wird.

## Claims

1. Balloon catheter comprising:
a balloon (1), wherein said balloon (1) has a balloon membrane made of one or more expandable elastomers; a conventional catheter shaft which forms a wire lumen which is designed to guide a guide wire, and forms a fluid lumen via which a fluid can be supplied to the balloon (1);
wherein said balloon (1) is attached to a first,
proximal attachment site (3) on a first catheter shaft member (5, 4', 5'); and
the balloon (1) is attached to a second, distal attachment site (2) on a second catheter shaft member (4, 12), wherein further the balloon (1) surrounds a portion of the catheter shaft that extends between the first and second attachment sites (3, 2);
and the portion of the catheter shaft which is surrounded by the balloon (1) comprises varying regions of the catheter shaft according to the changing longitudinal extension of the balloon (1);
**characterized in that**
the length of the balloon (1) in the unextended state is ≥ 10 cm, or with a diameter of 3 mm is ≥ 30 mm.

2. Balloon catheter according to Claim 1, wherein the second attachment site (2) is movable along a longitudinal axis of the catheter shaft relative to the first attachment site (3).

3. Balloon catheter according to Claim 1 or 2, wherein the one or more extendable elastomers are selected from rubber, latex, polyurethane, isoprene-based polymers or copolymers, silicone, particularly preferably from latex, polyurethane, or a copolymer of polystyrene and hydrogenated isoprene.

4. Balloon catheter according to any one of Claims 1-3, wherein the length of the balloon (1) in the unextended state is preferably ≥ 15 cm and particularly preferably ≥ 20 cm.

5. Balloon catheter according to any one of Claims 2-4, wherein the second catheter shaft member (12) is embodied as a guide element which is supported on the first catheter shaft member (4', 5') and is displaceable on the first catheter shaft member (4', 5') along a longitudinal axis of the first catheter shaft member (4', 5'), which forms the wire lumen.

6. Balloon catheter according to any one of claims 1 to 5, wherein with an internal pressure of <2.0266 bar (2 atm) above external pressure the diameter of the balloon is increased by a factor of at least 2.5, the balloon is extended in the longitudinal axis by at least 20% compared with its resting state, and after the attachment to the first and second catheter shaft members it remains under tension in the longitudinal axis, **characterized in that**
the elastic balloon material was pre-stretched by a factor of at least 3 perpendicularly to the longitudinal axis and is tension-free in this direction.

7. Balloon catheter according to any one of Claims 1 to 6, wherein the balloon (1) and/or the catheter shaft has/have physiologically compatible lubricants on the outer surfaces thereof.

8. Balloon catheter according to any one of Claims 1 to 7, wherein the balloon (1) is coated with at least one drug, preferably with at least one of the following drugs: paclitaxel, other taxanes, rapamycin, everolimus, zotarolimus, biolimus, betamethasone diproprionate, dexamethasone-21 palmitate,
atorvastatin, cerivastatin, fluvastatin, heparin, hirudin, acetylsalicylic acid, ticlopidine, clopidogrel, prasugrel, iloprost, prostaglandins E1, E2, F2α, warfarin, phenprocoumon.

9. Balloon catheter according to Claim 8, wherein the balloon (1) is coated with one or more drugs in a dosage between 100 µg/cm balloon length and 10 mg/cm balloon length, preferably 300-3000 µg/cm balloon length and particularly preferably 600-3000 µg/cm balloon length.

10. Balloon catheter according to any one of Claims 1 to 9, wherein the balloon (1) is coated with at least one antioxidant, preferably with at least one of the following antioxidants: ascorbic acid, ascorbyl palmitate, butylhydroxytoluene, butylated hydroxyanisole, nordihydroguaiaretic acid, propyl gallate, resveratrol, and/or nitro-oleic acid.

11. Balloon catheter according to Claims 1 to 10, wherein the balloon (1) undergoes prior treatment before use, **characterized in that** the balloon (1) is expanded at least once perpendicularly to its longitudinal axis over the entire length thereof with a factor of at least 1.1 relative to the diameter of the balloon (1) before expansion, without limitation of the expansion of the balloon (1) perpendicularly to the longitudinal axis.

12. Balloon catheter according to Claims 1 to 11, **characterized in that** in order to mount the balloon (1) on a conventional catheter shaft the balloon (1) is extended in the longitudinal axis by 20% to 120% relative to its resting state and is mounted on the catheter shaft while said extension is retained.

## Revendications

1. Cathéter à ballon comportant :
un ballon (1), le ballon (1) présentant une membrane à ballon qui est composée d'un ou plusieurs élastomères extensibles ; une tige de cathéter courante qui constitue un lumen à fil qui est conçu pour guider un fil de guidage, et constitue un lumen à liquide par lequel un liquide peut être acheminé au ballon (1) ;
le ballon (1) étant fixé par un premier point de fixation proximal (3) à un premier élément tige de cathéter (5, 4', 5') ; et
le ballon (1) étant fixé par un second point de fixation distal (2) à un second élément tige de cathéter (4, 12), le ballon (1) entourant en outre une section de la tige de cathéter qui s'étend entre les premier et second points de fixation (3, 2) ;
et la section entourée par le ballon (1) de la tige de cathéter entourant, par une extension de longueur variable du ballon (1), différentes parties de la tige de cathéter ; **caractérisé en ce que**
la longueur du ballon (1) en état non étiré est ≥ 10 cm ou ≥ 30 mm dans le cas d'un diamètre de 3 mm.

2. Cathéter à ballon selon la revendication 1, dans lequel le second point de fixation (2) est mobile par rapport au premier point de fixation (3) le long d'un axe longitudinal de la tige de cathéter.

3. Cathéter à ballon selon la revendication 1 ou 2, dans lequel le ou les plusieurs élastomères extensibles sont sélectionnés parmi le caoutchouc, le latex, le polyuréthane, les polymères ou copolymères à base d'isoprène, le silicone, très préférentiellement parmi le latex, le polyuréthane ou un copolymère de polystyrène et l'isoprène hydrogéné.

4. Cathéter à ballon selon l'une quelconque des revendications 1 à 3, dans lequel la longueur du ballon (1) en état non étiré est de préférence ≥ 15 cm et très préférentiellement ≥ 20 cm.

5. Cathéter à ballon selon l'une quelconque des revendications 2 à 4, dans lequel le second élément tige de cathéter (12) est réalisé sous forme d'un élément de guidage s'appuyant sur le premier élément tige de cathéter (4', 5') et qui est déplaçable le long d'un axe longitudinal du premier élément tige de cathéter (4', 5') constituant le lumen à fil sur le premier élément tige de cathéter (4', 5').

6. Cathéter à ballon selon l'une quelconque des revendications 1 à 5, dans lequel le ballon, avec une pression < 2,0266 bars (2 atm) agrandit son diamètre d'au moins 2,5 fois au-dessus de la pression extérieure, le ballon est étiré dans l'axe longitudinal d'au moins 20 % par rapport à son état au repos et, après sa fixation sur les premier et second éléments tiges de cathéter reste sous tension dans l'axe longitudinal, **caractérisé en ce que**
le matériau élastique du ballon a été préétiré perpendiculairement à l'axe longitudinal au moins à raison du facteur 3 et est dépourvu de tension dans ce sens.

7. Cathéter à ballon selon l'une quelconque des revendications 1 à 6, dans lequel le ballon (1) et/ou la tige de cathéter présente, au niveau de sa surface extérieure, des agents de glissement à compatibilité physiologique.

8. Cathéter à ballon selon l'une quelconque des revendications 1 à 7, dans lequel le ballon (1) est enduit d'au moins un médicament, de préférence d'au moins un des médicaments suivants : paclitaxel, autres taxanes, rapamycine, évérolimus, zotarolimus, biolimus, bétaméthasone dipropionate, dexaméthasone-21-palmitate, atorvastatine, cérivastatine, fluvastatine, héparine, hirudine, acide acétylsalicylique, ticlopidine, clopidogrel, prasugrel, iloprost, prostaglandine E1, E2, F2α, warfarine, phénprocoumone.

9. Cathéter à ballon selon la revendication 8, dans lequel le ballon (1) est enduit d'une ou plusieurs substances médicamenteuses dans une dose comprise entre 100 µg/cm et 10 mg/cm de longueur de ballon, de préférence 300 à 3000 µg/cm de longueur de ballon et très préférentiellement 600 à 3000 µg/cm.

10. Cathéter à ballon selon l'une quelconque des revendications 1 à 9, dans lequel le ballon (1) est enduit au moins un antioxydant, de préférence d'au moins un des antioxydants suivants : acide ascorbique, ascorbyl palmitate, butylhydroxytoluène, butylhydroxyanisol, acide nordihydrogénérateur, propylgallate, resvératrol, et/ou acide nitrooléique.

11. Cathéter à ballon selon les revendications 1 à 10, dans lequel le ballon (1) est traité avant usage, **caractérisé en ce que** le ballon (1) est dilaté au moins une fois sur toute sa longueur perpendiculairement à l'axe longitudinal d'au moins 1,1 fois par rapport au diamètre du ballon (1) avant dilatation, sans que l'étirement du ballon (1) perpendiculairement à l'axe longitudinal soit restreint.

12. Cathéter à ballon selon l'une quelconque des revendications 1 à 11, **caractérisé en ce que**, pour le montage du ballon (1) sur une tige de cathéter usuelle, le ballon (1) est étiré dans l'axe longitudinal de 20 % à 120 % par rapport à son état au repos et est monté sur la tige de cathéter en conservant cet étirement.
